Europäisches Patentamt

European Patent Office

Office européen des brevets

(11) Publication number: **0 481 160 A1**

# EUROPEAN PATENT APPLICATION

(21) Application number: **91105872.5**

(22) Date of filing: **12.04.91**

(51) Int. Cl.⁵: **A61L 33/00**

(30) Priority: **17.10.90 US 599129**

(43) Date of publication of application:
**22.04.92 Bulletin 92/17**

(84) Designated Contracting States:
**BE DE ES FR GB IT LU NL SE**

(71) Applicant: **CORVITA CORPORATION**
**P.O. Box 025700**
**Miami, Florida 33102-5700(US)**

(72) Inventor: **Pinchuk, Leonard, Dr.**
**9722 S.W. 133rd Place**
**Miami Florida 33186(US)**
Inventor: **Esquivel, Marie C.**
**6403 S.W. 42nd Street**
**Miami Florida 33155(US)**

(74) Representative: **KUHNEN, WACKER &**
**PARTNER**
**Alois-Steinecker-Strasse 22 Postfach 1553**
**W-8050 Freising(DE)**

(54) Antithrombogenic composition and methods of making same.

(57) An antithrombogenic composition comprising an anticoagulant bound to processed collagen. A method of preparing an antithrombogenic material useful in medical applications is provided wherein said the protein of processed collagen to which anticoagulant is bound is fixed by a cross-linking agent. A method of preparing an antithrombogenic material useful as a coating for conventional medical devices is provided wherein the device is impregnated with processed collagen, the protein of the processed collagen is fixed by a cross-linking agent, and the device as impregnated is treated with an anticoagulant and with a plasticizing agent.

The present invention generally relates to an antithrombogenic composition and methods for making same. More particularly, the invention relates to a composition from which a material may be made and that is particularly suitable for use within the body as a coating for medical devices. The composition includes an anticoagulant, such as heparin, whose antithrombogenic properties allows a material made from the composition or a device coated with the composition to be placed in direct contact with blood without inducing the resultant deleterious formation of thrombi.

Blood is held in a delicate balance within the body. Blood must be able to flow freely under normal circumstances. However, in response to an injury or the rupture of a blood vessel, blood must also be able to form a coagulated semisolid mass, a clot, that seals the body until tissue can grow back.

The chemistry of clotting is complex. Some six chemical reactions are required just to initiate the process. Some twelve factors from the plasma, the platelets, and tissues contribute to the process. The process begins with the conversion of a protein found in plasma, prothrombin, to thrombin. Thrombin autocatalyzes a protein found in soluble form in the plasma, fibrinogen, to an insoluble network of fibrous material, fibrin. Fibrin acts to reinforce the platelet aggregate to produce the final, stable clot. The terms clot and thrombus are largely interchangeable.

While clotting is a normal and wanted response to tissue injury, clotting is a harmful and unwanted response to beneficial procedures such as cardiovascular surgery. Presently, during such surgery, a variety of devices made from a variety of medical materials are implanted in the body generally to replace, or to provide support for tissue, organs, blood vessels and the like. The risk of associated complications and possible ultimate rejection are minimized when the source of the material is the patient. However, the medical status of the patient often does not allow the patient to act as a tissue and organ donor. More and more frequently, such devices are made from artificial materials. The artificial materials may be of natural origin - such as processed animal carotid arteries or human umbilical veins - or made from synthetic substances - such as a variety of biocompatible plastics - or a composite of both such materials. Because of the abundance of artificial materials, medical devices made from them are also readily available thereby allowing patients to undergo surgery despite their medical conditions. Artificial materials, and devices made from them, however, have a serious drawback: such materials often prompt a strong thrombogenic reaction when implanted in direct contact with blood in the host. In replacement or support devices implanted in blood vessels, this is a particularly serious problem as the resultant clotting ultimately prevents the flow of blood past the device.

A demand therefore is present for a composition from which a material can be made that can be used subcutaneously and in direct contact with blood but which does not prompt a strong thrombogenic reaction. The present invention satisfies the demand.

The present invention provides an antithrombogenic composition made from processed collagen and an anticoagulant. Depending upon the application, a material may be made from the composition that may be placed in direct contact with blood, and from which a medical device may be formed or from which, for example, a coating or impregnation may be made that is usable on a medical device as a means to suppress the formation of thrombi.

The present invention has a variety of advantages over conventional antithrombogenic materials. The composition of the present invention includes processed collagen to which is bound an anticoagulant. Conventional antithrombogenic materials generally use protamine as an agent to bind heparin to the material. The use of the added protamine component as a binding agent in conventional antithrombogenic materials is disadvantageous not only due to the greater time and expense in preparing the material, but also because protamine is an antagonist of heparin. As such, protamine acts to inactivate and block the anticoagulant effect of heparin. In fact, protamine is used in this antagonist capacity as a chemical antidote when a patient receives too great of a dose of heparin. Accordingly, medical material that utilizes protamine for a binding agent may have reduced or limited antithrombogenic properties. An additional advantage of the present invention is that it has been found that heparin bound to collagen as in the present invention remains bound for a far longer period of time (days) in comparison to conventional compositions. In comparison, it has been found that heparin bound by protamine in a conventional material, that is placed, for example, in a phosphate-buffered solution elutes from the material within a few hours. The elution of heparin from the conventional material is deleterious as free protamine can act as a clotting agent. An added advantage of the present invention is that anticoagulant is bound to processed collagen without a separate binding agent. Accordingly, the composition of the present invention is easier and less expensive to produce and the danger associated with the elution of the added binding agent - such as protamine - into the host's system is avoided.

It is accordingly a general object of the present invention to provide an antithrombogenic composition.

It is another object of the present invention to provide an antithrombogenic composition from which a material useable in medical applications may be made.

An additional object of the present invention is to provide a composition from which a medical material may be made that may be used as a coating or an impregnant.

A further object of this invention is to provide a method by which the composition may be applied to artificial material to form a device having antithrombogenic properties.

A further object of this invention is to provide a method by which a composition suitable for forming a device have antithrombogenic properties may be made.

These and other objects and advantages of the present invention will be clearly understood through a consideration of the following detailed description.

Description of the Particular Embodiments

The present invention provides a composition that includes processed collagen and a substance by which the formation of thrombi is prevented.

Collagen is a major extracellular structural protein found in connective tissue and bone. A number of advantages arise from such use of collagen as a source for one component of a composition from which biomedical materials are to be made. Certain types of collagen are highly biocompatible even when placed in direct contact with blood. In its natural setting, various forms of collagen are intimately involved in the circulation of blood. Collagen, in the form of fibers, is one part of the basement membrane that, for example, lines the lumens of the capillaries. Collagen also resists dissolution and, to a certain degree, chemical attack.

The preferred component by which the formation of thrombi may be prevented is heparin. Heparin is a naturally occurring substance formed and stored in the metachromatic granules of the mast cells located in the connective tissue surrounding capillaries and the walls of blood vessels. Heparin is particularly abundant in the liver and lung tissue. Heparin, obtained from sources such as beef liver or pig mucosa, is the clinical drug of choice to prevent the clotting of blood. Heparin inhibits clotting by combining with antithrombin to form a stable complex that increases the ability of antithrombin to inactivate thrombin. By inactivating thrombin, fibrinogen is prevented from forming fibrin, the very material from which the clot-supporting network is formed.

According to the present invention, the collagen may be processed to form gelatin by adding heat to or by boiling the collagen with water or acid. As denatured, the collagen protein forms gelatin. Collagen processed in the form of gelatin is also commercially available.

Processed collagen, in the form of gelatin may crack or otherwise become damaged during handling when used in a composition that is dried. Accordingly, to prevent such damage, the present invention may include a biocompatible plasticizing agent, such as glycerine and/or water.

It has been found that anticoagulant may be added to and bound within the processed collagen/elasticizing agent composition without protamine. While not wishing to be bound by any specific theory, it is believed that, given the hydrophilic nature of processed collagen and the hydrophilic nature of an anticoagulant such as heparin, the anticoagulant is bound to the processed collagen by hydrogen bonds. A hydrogen bond is the attractive force, or bridge that occurs in polar compounds, such as water, in which a hydrogen atom is attracted to two electrons of different atoms. Alternatively, the attraction between the processed collagen and the anticoagulant may be the result of Van der Waals' forces. The attraction further alternatively between the processed collagen and the anticoagulant may be the result of covalent bonding between amine groups on heparin and residual aldehyde groups on glutaraldehyde. The attraction may be the result also of ionic bonding between the negatively-charged sulphates on heparin and the positively-charged amine groups on collagen.

The composition is suitable for forming a material having a desired configuration. The material is chilled and immersed in a solution containing an agent by which the protein of the processed collagen is cross-linked. While processed collagen is generally soluble at elevated temperatures, cross-linking stabilizes the material so that it may remain in the body for an extended period of time. Suitable crosslinking agents can include glycidyl-terminated polyethylene oxide, glutaraldehyde, formaldehyde, isocyanate-terminated polyethylene oxides, or the like. However, glutaraldehyde is a preferred cross-linking agent.

A medical material, according to the present invention, usable as a coating or impregnant for conventional medical materials and devices, or by which medical devices may be made, and by which the formation of thrombi is prevented, may be made as follows. The conventional medical device may be, for example, a porous vascular graft made from synthetic material or material of a natural origin or a composite of both. Specifically with regard to such a conventional vascular graft, the graft may be immersed in a liquid solution, preferably of phosphate buffered saline warmed to 50°C, containing processed collagen. Typically, a vacuum is applied in order to impregnate the graft with the processed collagen. Excess processed collagen is removed from the graft by dripping, and the graft containing the gelatin is chilled to 4°C for 60 minutes causing the collagen to gel. The graft with the gelled collagen is then immersed in a chilled phosphate buffered saline solution including glutaraldehyde and crosslinked. The graft is then well rinsed in distilled water. The coated graft is then incubated in a solution bath, preferably a buffered acidic bath having an approximate pH of 3, containing an anticoagulant such as heparin. While the percentage of heparin in the bath may range from .001% to 10%, the preferred concentration is approximately 2%. The heparin and collagen containing graft is then again rinsed and finally plasticised by immersing it in a 10% solution of glycerine. The graft is then air dried and packaged.

When used as a coating on a graft of synthetic materials, the medical material of the present invention provides a number of added advantages. It is conventionally necessary to hydrate such grafts prior to use. The medical material of the present invention aids in the hydration process. Gelatin is strongly hydrophilic and absorbs some ten times its weight in water. Similarly, an anticoagulant, such as heparin, is also a hydrophilic substance, particularly because of its sulfate groups and assists in the rehydration.

It has been found that the three month patency of a medical device coated or impregnated with the material is at least equal to that material including protamine. Such a protamine-free device, however, does not have the associated problems of short-term elution of heparin from the material and the potential clotting caused by the protamine.

It will be understood that the embodiments of the present invention which have been described are illustrative of some of the applications of the principles of the present invention. Numerous modifications may be made by those skilled in the art without departing from the true spirit and scope of the invention.

## Claims

1. An antithrombogenic medical composition comprising a substantially protamine-free composition containing collagen and an anticoagulant bound to said collagen whereby said collagen is cross-linked to form a medical material useful in medical applications without inducing a thrombogenic reaction.

2. The antithrombogenic medical composition according to claim 1, wherein said anticoagulant is bound to said collagen by hydrogen bonds.

3. The antithrombogenic medical composition according to claim 1, wherein said anticoagulant is heparin.

4. The antithrombogenic composition according to claim 3, wherein said anticoagulant is bound to said collagen by hydrogen bonds.

5. The antithrombogenic composition according to claim 3, wherein said anticoagulant is bound to said collagen by Van der Waals' forces.

6. The antithrombogenic composition according to claim 3, wherein said anticoagulant is bound to said collagen by ionic bonds.

7. The antithrombogenic composition according to claim 3, wherein said anticoagulant is bound to said collagen by covalent bonds.

8. The antithrombogenic medical composition according to claim 1, wherein said collagen is processed to form gelatin.

9. The antithrombogenic medical composition according to claim 1, wherein said cross-linking agent is glutaraldehyde.

10. The antithrombogenic medical composition according to claim 1, further including a plasticizing agent to prevent damage to the material during handling.

11. The antithrombogenic medical composition according to claim 10, wherein said plasticizing agent is glycerine.

12. A composition useful as an antithrombogenic coating for a medical device comprising gelatin impregnated into a shaped medical device and an anticoagulant absorbed from a solution by and bound to said gelatin whereby said gelatin is fixed by a cross-linking agent to provide a coated device that is substantially free of protamine and is useful in medical applications without inducing a thrombogenic reaction.

13. The composition according to claim 12, wherein said anticoagulant is bound to said gelatin by hydrogen bonds.

14. The composition according to claim 12, wherein said anticoagulant is bound to said gelatin by Van der Waals' forces.

15. The composition according to claim 12, wherein said anticoagulant is bound to said gelatin by ionic bonds.

16. The composition according to claim 12, wherein said anticoagulant is bound to said gelatin by covalent bonds.

17. The composition according to claim 12, wherein said anticoagulant is heparin.

18. The composition according to claim 12, wherein said cross-linking agent is glutaraldehyde.

19. The composition according to claim 12, wherein damage to said gelatin is prevented by a plasticizing agent.

20. The composition according to claim 19, wherein said plasticizing agent is glycerine.

21. A method of preparing an antithrombogenic material substantially free of protamine and useful in medical applications comprising processing collagen to form gelatin, treating the gelatin with an agent to cross-link protein of said gelatin, adding an anticoagulant to said gelatin whereby said anticoagulant is bound to said gelatin, and treating said gelatin with a plasticizing agent.

22. The method according to claim 21, wherein said anticoagulant is heparin.

23. The method according to claim 21, wherein said plasticizing agent is glycerine.

24. The method according to claim 21, wherein said cross-linking agent is glutaraldehyde.

25. A method of coating a device shaped for and useful in medical applications without the inducement of a thrombogenic reaction, said method comprising impregnating a device shaped for medical applications with collagen denatured to form gelatin, fixing said gelatin by a cross-linking agent, treating said device with an anticoagulant whereby said anticoagulant is bound to said gelatin and treating said device impregnated with said gelatin with a plasticizing agent.

26. The method according to claim 25, wherein said anticoagulant is heparin.

27. The method according to claim 25, wherein said plasticizing agent is glycerine.

28. The method according to claim 25, wherein said cross-linking agent is glutaraldehyde.

29. The method according to claim 25, wherein said heparin is bound to said gelatin by hydrogen bonds.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 246 638 (CORDIS) | 1-7,9 | A 61 L 33/00 |
| Y | (* claims 15,26,30; example 1 *) | 8,10-29 | |
| | – – – | | |
| X | EP-A-0 241 838 (SUMITOMO ELECTRIC) | 1 | |
| | * claims 1-4 * * | | |
| | – – – | | |
| Y | GB-A-2 166 977 (MITSUBISHI MONSANTO) | 8,10-29 | |
| | * claims 6,8,9 * * | | |
| | – – – | | |
| A | EP-A-0 237 037 (INTERMEDICAT) | 12 | |
| | * claims 9,12 * * | | |
| | – – – – – | | |

| | | TECHNICAL FIELDS SEARCHED (Int. Cl.5) |
|---|---|---|
| | | A 61 L |

The present search report has been drawn up for all claims

| Place of search | Date of completion of search | Examiner |
|---|---|---|
| The Hague | 23 January 92 | PELTRE CHR. |